# EUROPÄISCHE PATENTSCHRIFT

(11) **EP 1 051 619 B1**
(45) Veröffentlichungstag und Bekanntmachung des Hinweises auf die Patenterteilung: **09.08.2006**
(21) Anmeldenummer: 99930888.5
(22) Anmeldetag: 15.01.1999
(51) Int. Cl.: G01N 33/50, G01N 33/68, C12P 21/00, A61K 38/17

(54) **VERFAHREN ZUM IDENTIFIZIEREN VON T-ZELL-STIMULIERENDEN PROTEINFRAGMENTEN**
METHOD FOR IDENTIFYING T-CELL STIMULATING PROTEIN FRAGMENTS
PROCEDE D'IDENTIFICATION DE FRAGMENTS DE PROTEINE STIMULANT LES LYMPHOCYTEST

(30) Priorität: 19.01.1998 DE 19802174; 28.07.1998 DE 19834932
(43) Veröffentlichungstag der Anmeldung: 15.11.2000
(73) Patentinhaber: Kern, Florian, 10435 Berlin (DE)
(72) Erfinder: VOLK, Hans-Dieter, 10178 Berlin (DE); WALDEN, Peter, 10405 Berlin (DE); SCHEFFOLD, Alexander, 10969 Berlin (DE); BLASCZYK, Rainer, 30989 Burgwedel (DE); KERN, Florian, 10435 Berlin (DE)
(74) Vertreter: Helbing, Jörg
(86) Internationale Anmeldenummer: PCT/DE1999/000175
(87) Internationale Veröffentlichungsnummer: WO 1999/036568

(56) Entgegenhaltungen:
- WOITAS RP, LECHMANN M, JUNG G, KAISER R, SAUERBRUCH T, SPENGLER U: "CD30 induction and cytokine profiles in hepatitis C virus core-specific peripheral blood T lymphocytes" JOURNAL OF IMMUNOLOGY, Bd. 159, Nr. 2, 15. Juli 1997 (1997-07-15), Seiten 1012-1018, XP002125046
- STEPANIAK, JOLIE A. ET AL: "A comparative study of experimental autoimmune encephalomyelitis in Lewis and DA rats" J. IMMUNOL. (1995), 155(5), 2762-9 , XP002125047
- SURCEL HM, TROYE-BLOMBERG M, PAULIE S, ANDERSSON G, MORENO C, PASVOL G, IVANYI J: "Th1/Th2 profiles in tuberculosis, based on the proliferation and cytokine response of blood lymphocytes to mycobacterial antigens" IMMUNOLOGY, Bd. 81, Nr. 2, Februar 1994 (1994-02), Seiten 171-176, XP000863031
- AUSUBEL LJ, KRIEGER JI, HAFLER DA: "Changes in cytokine secretion induced by altered peptide ligands of myelin basic protein peptide 85-99" JOURNAL OF IMMUNOLOGY, Bd. 159, Nr. 4, 1. September 1997 (1997-09-01), Seiten 2502-2512, XP002125048
- CELLO J, STRANNEGARD O, SVENNERHOLM B: "A study of the cellular immune response to enteroviruses in humans: identification of cross-reactive T cell epitopes on the structural proteins of enteroviruses" JOURNAL OF GENERAL VIROLOGY, Bd. 77, Nr. 9, Januar 1996 (1996-01), Seiten 2097-2108, XP002125049
- PICKER LJ ET AL: "Direct demonstration of cytokine synthesis heterogeneity among human memory/effector T cells by flow cytometry" BLOOD,US,PHILADELPHIA, PA, Bd. 86, Nr. 4, Seite 1408-1419-1419 XP002108552 ISSN: 0006-4971 in der Anmeldung erwähnt
- KERN F; SUREL I P; FAULHABER N; FROMMEL C; SCHNEIDER-MERGENER J; SCHONEMANN C; REINKE P; VOLK H D: "Target structures of the CD8(+)-T-cell response to human cytomegalovirus: the 72-kilodalton major immediate-early protein revisited" JOURNAL OF VIROLOGY, Bd. 73, Nr. 10, Oktober 1999 (1999-10), Seiten 8179-8184, XP002125050

## Beschreibung

### Stand der Technik

Die T-Zell-stimulierenden Proteinfragmente umfassen T-Zell-Epitope, die von T-Zell-Rezeptoren spezifisch erkannt werden und mittels dieser Erkennung unter anderem die T-Zellen zur Biosynthese von Zytokinen, die üblicher Weise sekretiert werden, anregen.

Ein bekanntes Verfahren zur Identifizierung von T-Zell stimulierenden Proteinfragmenten besteht darin, daß ein Protein, dessen Aminosäure-Sequenz bekannt ist, in einzelne überlappende Proteinfragmente aufgeteilt wird. Die entsprechenden synthetisch hergestellten Proteinfragmente werden einzeln oder in Gruppen mit T-Zellen inkubiert. Nach ein bis drei Wochen liegen gegebenenfalls Zell-Linien oder Zell-Klone vor, die spezifisch durch das bzw. mindestens eines der eingesetzten Proteinfragment stimuliert werden konnten. Die Spezifität dieser Linien oder Klone kann durch Zytotoxizitatstestung an ensprechenden Zielzellen (engl.: target cells) nachgewiesen werden. Aufgrund der Versuchsanordnung können die stimulierten Zell-Linien oder Zell-Klone den entsprechenden T-Zell stimulierenden Proteinfragmenten zugeordnet werden. Diese Methode ist ausführlich in P. WALDEN et al. (1996) Current Opinion in Immunology, Vol. 8, pp 68-74 beschrieben. Alternativ kann die Proliferation von Zellen nach 1 Woche durch Inkorporation von 3H-Thymidin bestimmt werden, was aber mit größerer Unspezifität behaftet ist. Nachteil dieser beiden Methoden ist der hohe apparative, personelle und zeitliche Aufwand. Außerdem ist es wahrscheinlich, daß stimulierte T-Zellen während der langen Inkubationszeit absterben, z.B. durch den aktivierungsinduzierten, programmierten Zelltod (Apoptose) und falsch negative Ergebnisse daraus resultieren.

Ein Verfahren zur durchflußzytometrischen Identifizierung von Antigen-spezifischen T-Zellen nach S. L. WALDROP et al., (1997) Determination of antigen-specific memory/effector CD4+ T cell frequencies by flow cytometry: evidence for a novel antigen-specific homeostatic mechanism in HIV-associated immunodeficiency. J Clin. Invest. Vol 99, pp 1739-1750 besteht darin, daß Proteine als Antigen mit peripheren mononukleären Zellen (PBMC = peripheral blood mononuclear cells) inkubiert werden. Dabei werden diese Proteine von Antigen-präsentierenden Zellen prozessiert und präsentiert. Diese Prozessierung führt zu Proteinfragmenten, mit welchen MHC-Klasse-II-Moleküle beladen werden und dann zur Zelloberfläche gelangen (Antigenpräsentation). Die durch die Erkennung von Proteinfragmenten jeweils stimulierten T-Zellen werden durchflußzytometrisch identifiziert. Dabei ist es weder Möglich die stimulierenden Proteinframente zu ermitteln, noch die spezifisch induzierten T-Zellen den induzierenden Proteinfragmenten zuzuordnen. Aufgabe dieser Versuchsanordnung ist es vor allem, festzustellen, ob MHC-Klasse-II präsentierte Epitope in einem Protein oder komplexen Antigen vorhanden sind bzw. ob ein Individuum gegen solche möglicherweise oder bekanntermaßen vorhandenen Epitope spezifische MHC-Klasse-II restringierte T-Zellen besitzt und wie hoch die Frequenz dieser Zellen ist (Quantifizierung der antigenspezifischen T-Zellen). Zusätzlich lassen sich weitere Eigenschaften der stimulierten T-Zellen ermitteln (Oberflächenmarker etc.). Aber, weder die Aminosäure-Sequenz vorhandener Epitope noch die Häufigkeit solcher Epitope läßt sich ermitteln.

Weiterhin beschreibt R. P. Woitas in J. Immunol. 159(2):1012-1018 (1997) die Induktion von CD30 und Cytokinen durch die Wirkung des HCV Core Proteins oder Fragmente dessen auf periphere mononukleäre Zellen von Hepatitis Patienten. Die Inkubationsdauer der Peptide mit den Zellen beträgt 40 h. Die T-Zell Cytokine wie z. B. IL-2 und IFN-γ werden durchflusszytometrisch und damit auf Einzelzell-Ebene bestimmt.

### Aufgabe und Lösung

Es ist daher Aufgabe der Erfindung, ein Verfahren anzubieten, mit dem Proteinfragmente, deren Aminosäure-Sequenzen bekannt sind, in kurzer Zeit als stimulierende Proteinfragmente identifiziert werden können. Dabei soll die Methode auch bei kleiner Anzahl an T-Zellen arbeiten, ohne daß T-Zell-Linien oder -Klone zur Verfügung stehen müssen. Weiterhin soll es möglich sein, aus einer großen Anzahl an Proteinfragmenten, diejenigen herauszufinden, welche T-Zellen stimulieren.

Die Aufgabe wird gelöst durch ein Verfahren zum Identifizieren von T-Zell-stimulierenden Proteinfragmenten, die folgenden Schritte umfassend:
a) Ermitteln der Aminosäuresequenz eines Antigens, welches ein Protein oder Peptid ist,
b) Unterteilen der gefundenen Aminosäuresequenz des Antigens in Proteinfragmente,
c) Synthetisieren von mindestens einem Proteinfragment mit einer Länge von 8 bis 30 Aminosäuren oder Spalten der Aminosäuresequenz des Antigens zu mindestens einem Proteinfragment mit einer Länge von 8 bis 30 Aminosäuren,
   dabei ist das Proteinfragment eine Teilsequenz der ermittelten Aminosäuresequenz des Antigens,
d) Inkubieren einer T-Zellen enthaltenden Suspension mit dem oder den Proteinfragmenten in Versuchsansätzen,
e) Identifizieren
   (i) von mindestens einem T-Zell-Zytokin, das durch das oder die Proteinfragmente induziert und in den T-Zellen synthetisiert wurde, dabei liegen das oder die T-Zell-Zytokine intrazellulär oder an die Zellmembran gebunden vor, und / oder
   (ii) von mindestens einem Aktivierungsmarker, der durch das oder die Proteinfragmente induziert oder in seiner Expression gesteigert wurde und in den T-Zellen exprimiert wird,
      dabei kann der Aktivierungsmarker intrazellulär vorliegen oder auf der Zelloberfläche exprimiert sein
   dabei werden das oder die T-Zell-Zytokine oder Aktivierungsmarker durchflußzytometrisch identifiziert, und
f) Zuordnen der Versuchsansätze, bei denen T-Zellen stimuliert wurden und diese T-Zell-Stimulation durch das Identifizieren von einem T-Zell-Zytokin oder mehreren T-Zeil-Zytokinen und / oder einem oder mehreren Aktivierungsmarkern erkannt wurde, zu der oder den Aminosäuresequenzen der Proteinfragmente, welche mit den T-Zellen inkubiert wurden, dadurch gekennzeichnet, dass die Inkubationszeit so lang ist, dass das oder die Proteinfragmente von den MHC Molekülen, die sich auf der Zelloberfläche befinden, ausreichend aufgenommen werden, wobei die Aufnahme dann ausreichend ist, wenn eine eindeutige Identifizierung stimulierter T-Zellen möglich ist, und, dass die Inkubationszeit der T-Zellen enthaltenden Suspension mit dem oder den Proteinfragmenten so kurz ist, dass eine Selektion oder Proliferation, die mit einer gezielten Eliminierung bestimmter T-Zellen einhergeht, nicht erfolgt.

### Vorteile:

Der Vorteil dieses erfindungsgemäßen Verfahrens besteht darin, daß innerhalb von sehr kurzer Zeit und im Vergleich zur konventionellen Methode mit sehr geringem Aufwand ein bezüglich der Sequenz bekanntes Proteinfragment als ein T-Zell stimulierendes Proteinfragment identifiziert werden kann. Die Zeit zwischen erster Inkubation von T-Zellen und durchflußzytometrischer Auswertung kann sechs Stunden betragen. Dabei können kleinste Zell-Zahlen ausreichen. Wenn mit einer Anzahl von 1 • 10⁶ peripheren weißen Blutzellen gestartet wird, kann zweifelsfrei eine positive Antwort noch festgestellt werden, wenn 0,1% der Ausgangs-T-Zellzahl stimulierte T-Zellen sind. Dagegen benötigt die klassische Methode eine Zellzahl von etwa 8 • 10⁶ peripheren weißen Blutzellen je Proteinfragment oder Mischung aus Proteinfragmenten, um anschließend einen Zytotoxizitätstest erfolgreich durchführen zu können. Das erfindungsgemäße Verfahren ist also ein Verfahren, welches mit hoher Effizienz zum T-Zell-Epitopmapping von Proteinantigenen eingesetzt werden kann.

Weiterhin können Gemische aus frisch isolierten zellulären Blutzellen oder Gewebezellen verwendet werden. T-Zell-Linien oder T-Zell-Klone sind nicht für dieses erfindungsgemäße Verfahren notwendig. Hierdurch ergeben sich Zeitvorteile bei der Inkubation und weiterhin sehr wesentlich, ein Vorteil bezüglich der Viabilität der T-Zellen, welche in der kurzen Inkubationszeit als großer Pool mit hoher Variabilität vorliegen. Eine Selektion und Proliferation, die mit einer gezielte Eliminierung bestimmter T-Zellen einhergeht, erfolgt aufgrund der kurzen Inkubationszeiten bei dem erfindungsgemäßen Verfahren nicht.

Bevorzugt als Quelle der zu stimulierenden T-Zellen sind solche Spender, welche zuvor eine immunologische Primärantwort gegen das Antigen aufgebaut haben. Dies kann beispielsweise im Rahmen einer Infektion stattgefunden haben oder auch im Rahmen einer Immunisierung. Auch bei einer Autoimmunantwort ist diese Situation gegeben.

Ein weiterer Vorteil besteht darin, daß der MHC-Typ des Spenders nicht bekannt sein muß. So werden zum Beispiel Proteinfragmente mit 9 Aminosäuren aus einem Protein mit den T-Zellen inkubiert, ohne daß man den MHC-Typ des Blut-oder Zellspenders kennt. Dennoch lassen sich die T-Zell stimulierenden Proteinfragmente identifizieren. Somit ist zum Identifizieren des Epitops die Kenntnis des MHC-Typs nicht erforderlich. Beim klassischen Test mittels zytotoxischen T-Zell-Linien oder Klonen müssen die Zielzell-Linien (Target-Zell-Linien) im MHC mit den Effektor-Zellen übereinstimmen.

Das Erstellen von Target-Zell-Linien aus Spenderblut bedeutet einen zusätzlichen materiellen und zeitlichen Aufwand.

Weiterhin kann mit dem erfindungsgemäßen Verfahren eine große Anzahl an Proteinfragmenten zur selben Zeit inkubiert werden. Geringe Zell-Zahlen und hochsensitive Detektion stimulierter T-Zellen erlauben eine zeitlich deutlich vorteilhafte Identifizierung der T-Zell stimulierenden Proteinfragmente.

Da die Anzahl der zu untersuchenden Proteinfragmente aufgrund des geringen notwendigen Arbeitsaufwandes sehr hoch sein kann, ist es nicht notwendig mögliche Epitope mittels theoretischer Vorhersagen einzugrenzen. Die Epitope werden rein empirisch gefunden, und es können deshalb auch solche T-Zell-Epitope gefunden werden, die sich aufgrund einer theoretischen Voraussage nicht ergeben würden.

Mit diesem Verfahren lassen sich leicht T-Zellen identifizieren, die spezifisch durch bestimmte ausgewählte Proteinfragmente stimulierbar sind.

T-Zell-stimulierende Proteinfragmente binden einerseits an definierte MHC-Moleküle und andererseits enthalten sie Aminosäuresequenzen (Epitope), welche mit der Antigenbindungsregion des T-Zell-Rezeptors (Paratop) eine Bindung eingehen können.

Die Begriffe Protein oder Peptid haben als wesentliches Merkmal die Sequenz von mindestens neun Aminosäuren. Dabei ist gleichgültig, wie die Sequenz ermittelt worden ist. So kann bei einem neuen Protein die Sequenz zum erstenmal analysiert werden oder bei bekannten Protein aus einer Datenbank abgelesen werden. Wichtig ist nur, daß die Aminosäuresequenz des Proteinfragments bestimmt ist. Auch die Unterteilung der Protein-oder Peptidsequenz kann unterschiedlich ausfallen. So können die Proteinfragmente schrittweise mit der Variation von einer Aminosäure aus einem Protein abgeleitet werden. Andere Überlappungen sind ebenfalls denkbar. Es handelt sich dabei um das klassische Verfahren eines Protein-Mappings.

T-Zellen enthaltende Suspensionen im Sinne dieser Anmeldung zeichnen sich dadurch aus, daß sie Zellen enthalten, welche MHC-gebundene Peptide präsentieren können. So können die präsentierenden Zellen neben den Antigen-präsentierenden Zellen auch zum Beispiel T-Zellen sein.

### Weitere Ausführungsformen

Vorteilhaft ist das erfindungsgemäße Verfahren zum Identifizieren von T-Zell-stimulierenden Proteinfragmenten, da das Identifizieren von mindestens einem

T-Zell-Zytokin oder Aktivierungsmarker auf der Einzelzell-Ebene erfolgt. Schon kleinste Mengen an T-Zellen, welche Zytokine intrazellulär oder an die Zellmembran gebunden enthalten, reichen aus.

Bevorzugt ist ein erfindungsgemäßes Verfahren zum Identifizieren von T-Zell-stimulierenden Proteinfragmenten, bei dem die T-Zellen enthaltenden Suspensionen Zellen enthalten, die das Proteinfragment im wesentlichen mit MHC-Klasse-I oder-II (Haupt Histokompatibillitäts Komplex, MHC = Major Histocompatibility Complex) präsentieren. Neben den zur Verankerung in der Spalte des MHC-Moleküls dienenden Aminosäuren (Bindungsanker) müssen bestimmte Sequenzen vorhanden sein, die von einem T-Zell-Rezeptor spezifisch erkannt werden (T-Zell-Epitope), damit das Proteinfragment als T-Zell-Epitop funktioniert.

Bevorzugt ist ein erfindungsgemäßes Verfahren zum Identifizieren von T-Zell-stimulierenden Proteinfragmenten, bei dem das Proteinfragment bei der Klasse I restringierten Präsentation 9 bis 11 Aminosäuren umfaßt und das Proteinfragment bei der Klasse II restringierten Präsentation mindestens 11 Aminosäuren umfaßt. Es ist bekannt, daß an Moleküle der MHC-Klasse I (MHC = Major Histocompatibility Complex) bindende Proteinfragmente in der Regel eine Länge von 9 Aminosäuren aufweisen, während Proteinfragmente, welche an MHC-Klasse II Moleküle binden, etwas länger und in der Länge stärker variabel sind.

Vorteilhaft ist, daß die Proteinfragmente trotz der kurzen Inkubationszeit von den MHC-Molekülen, die sich auf der Zelloberfläche befinden, ausreichend aufgenommen werden, um eine eindeutige Identifizierung stimulierter T-Zellen nach zum Beispiel sechs Stunden zu ermöglichen. Werden weiterhin kurze Proteinfragmente (Klasse I mit 9 Aminosäuren und Klasse II mit vorzugsweise 11-15 Aminosäuren) verwendet, läßt sich das in einer stimulierenden Aminosäuresequenz vorhandene Epitop maximal eingrenzen.

Bevorzugt ist ein erfindungsgemäßes Verfahren zum Identifizieren von T-Zell-stimulierenden Proteinfragmenten, bei dem die T-Zellen enthaltende Suspension eine Suspension ist aus Vollblut, peripheren weißen Blutzellen (PWBC), Milzzellen, Thymuszellen, Knochenmark, Liquor und / oder aus Lymphknotenzellen. Das Verfahren wird erheblich dadurch vereinfacht, daß die T-Zellen enthaltenden Suspensionen aus unterschiedlichster Quelle stammen können. Weiterhin ist besonders vorteilhaft, daß eine Aufarbeitung der T-Zellen nicht erforderlich ist. So müssen die T-Zellen nicht angereichert werden, weiterhin ist ein Entfernen oder Zerstören von anderen Zellen nicht notwendig. Hierdurch läßt sich das erfindungsgemäße Verfahren einfacher routinemäßig handhaben. Das Verfahren ist nicht so störanfällig durch Kulturbedingungen, Kontaminationen, kulturbedingte Selektionen und Selektionierung von spezifischen Klonen wie das konventionelle Verfahren. Ein repräsentatives Bild von T-Zellen allgemein und T-Zellen, die durch Proteinfragmente stimuliert werden, läßt sich mit diesem Verfahren ermitteln.

Bevorzugt ist ein erfindungsgemäßes Verfahren zum Identifizieren von T-Zell-stimulierenden Proteinfragmenten, bei dem die T-Zellen enthaltende Suspension aus den Patienten, die therapiert werden sollen, aus Spendern oder aus Tieren stammen. Stammt die T-Zellen enthaltende Suspension aus einem Patienten, so läßt sich mit der Identifizierung zum Beispiel feststellen, gegen welches Proteinfragment/Epitop eines Virus-Antigens sich eine T-Zell-Antwort induzieren läßt. Ein solches Proteinfragment/Epitop kann dann zur Stimulation weiterer T-Zellen des Patienten gezielt eingesetzt werden. Die so induzierten und zur Proliferation angeregten Zellen können so expandiert und anschließend dem Patienten retranfusioniert werden. Das erfindungsgemäße Verfahren läßt sich auch in der Tiermedizin verwenden. Dabei sind unterschiedlichste Tierarten und auch Konstellationen von Tierpatienten und Spendern als Quelle der T-Zellen enthaltenden Suspension denkbar.

Vorteilhaft ist ein erfindungsgemäßes Verfahren zum Identifizieren von T-Zell-stimulierenden Proteinfragmenten, bei dem die Antigene, welche Proteine oder Peptide sind, aus Mikroorganismen, aus Makroorganismen, aus Zellen, Zellkulturen und / oder Geweben von Spendern oder Patienten stammen. Mikroorganismen sind zum Beispiel Viren, Bakterien, Pilze, Einzeller, Parasiten. Unter Makroorganismen fallen zum Beispiel alle mehrzelligen Eukaryoten. Gerade diese Quelle ist für die Beeinflussung von Allergien wichtig. Hierunter fallen Tiere und Pflanzen. Es können Zellen, Zellkulturen oder auch ganze Gewebe bestehend aus einer oder mehreren Schichten oder Zell-Typen verwendet werden.

Bevorzugt ist ein erfindungsgemäßes Verfahren zum Identifizieren von T-Zell-stimulierenden Proteinfragmenten, bei dem die T-Zell-Zytokine vom Typ Interferon-γ, TNF-α (Tumor-Nekrose-Faktor-alpha) oder Interieukin 2 sind. Jedoch sind auch andere Zytokine möglich. Hier ist allein von Bedeutung, daß diese Zytokine fluoreszenzmarkiert werden können.

Auch können Aktivierungsmarker identifiziert werden, die aufgrund der T-Zell-Stimulation durch die Proteinfragmente exprimiert oder in der Expression gesteigert werden. Der Marker CD69 ist hierfür beispielhaft. Beim Identifizieren von Aktivierungsmarkern die sich auf der Zelloberfläche befinden oder nicht sekretiert werden ist gegebenenfalls die Inhibition der Sekretion nicht mehr erforderlich. Zytokine und Oberflächenmarker sind ausführlich beschrieben in Abul K. ABBAS et al. (1997) Cellular and Molecular Immunology, Philadelphia, 3. Auflage, ISBN 0-7216-4024-9.

Mehr bevorzugt ist ein erfindungsgemäßes Verfahren zum Identifizieren von T-Zell-stimulierenden Proteinfragmenten, bei dem die T-Zell-Zytokine nach einer Inhibition der Sekretion intrazellulär vorliegen. Bedeutsam ist, daß die erfolgte Stimulation eindeutig T-Zellen zuzuordnen ist.

Bevorzugt ist ein erfindungsgemäßes Verfahren zum Identifizieren von T-Zell-stimulierenden Proteinfragmenten, wobei die Stimulation mittels eines Durchflußzytometers erfaßt wird Wesentlich ist dabei das Prinzip, daß Marker, die sich in der Zelle oder auf deren Oberfläche befinden, wie beispielsweise Zytokine oder Oberflächenmarker mit einem spezifischen Detektor, zum Beispiel einem Antikörper in Kontakt treten, wobei der Detektor mit einem Fluoreszenzfarbstoff beladen ist. Nach Anregung dieses Fluoreszenzfarbstoffes auf den in einem Flüssigkeitsstrom fokussierten Zellen durch Laserlicht zeichnet das Durchflußzytometer die emittierten Streulicht und Fluoreszenzsignale auf, was die zeitgleiche oder spätere Analyse der Zellen ermöglicht. Ausführlich sind solche Techniken beschreiben in Howard M. SHAPIRO (1995) Practical Flow Cytometry, New York, 3. Auflage, ISBN 0-471-30376-3. Die Detektion der intrazellulären Zytokine ist beschrieben in L J. PICKER et al. (1995) Blood, Vol. 86, pp 1408.

### Herstellung von T-Zell-stimulierenden Proteinfragmenten

Das erfindungsgemäße Verfahren umfasst weiterhin die Herstellung eines Proteinfragmentes/Peptides, das T-Zell-stimulierend ist und dessen Aminosäuresequenz oder ausgängliche Aminosäuresequenz nach dem erfindungsgemäßen Verfahren zum Identifizieren von T-Zell-stimulierenden Proteinfragmenten gefunden worden ist, wobei das Proteinfragment/Peptid mit der Festphasenmethode, der Flüssigphasenmethode oder mittels der Proteinbiosynthese in einem Wirt hergestellt wird.
Festphasen-Synthese: Die Festphasen-Synthese ist ausführlich beschreiben in Solid Phase Synthesis, E. ATHERTON and R.C. SHEPPARD (1989) IRL Press, ISBN 1-85221-133-4 und Amino Acid and Peptide Syntheses, J. JONES, Oxford Science Publication (1992) ISBN 0-19-855668-3.
Flüssigphasen-Synthese: Die Flüssigphasen-Synthese oder Lösungstechnik ist in Methoden der Organischen Chemie (HOUBEN/WEYL), Bd. 15 / Nr. 1 und 2, E. WÜNSCH (Herausgeber), Thieme Verlag Stuttgart, 1974 dargestellt.

Vorteilhaft ist weiterhin ein Verfahren zur Herstellung eines Proteinfragmentes/Peptides, das T-Zell-stimulierend ist und dessen Aminosäuresequenz oder ausgängliche Aminosäuresequenz nach dem erfindungsgemäßen Verfahren zum Identifizieren von T-Zell-stimulierenden Proteinfragmenten gefunden worden ist, wobei das Proteinfragment/Peptid mit der Festphasenmethode, der Flüssigphasenmethode oder mittels der Proteinbiosynthese in einem Wirt hergestellt wird, dabei weist das Proteinfragment/Peptid Insertionen, Deletionen oder Substituierungen auf (Modifikationen), wobei eine, zwei, drei oder mehrere Aminosäuren ausgetauscht, deletiert oder inseriert sind,
wobei das modifizierte Proteinfragment/Peptid im wesentlichen dieselbe Funktion bezüglich der Stimulation von T - Zellen aufweist, die das nicht modifizierte Proteinfragment/Peptid besitzt.

Besonders vorteilhaft ist ein Verfahren zur Herstellung eines Proteinfragmentes/Peptides der vorherigen Art,
wobei das Proteinfragment / Peptid am N-terminalen und / oder C-terminalen Ende mindestens eine weitere natürliche oder nichtnatürliche Aminosäure und / oder eine Schutzgruppe besitzt (erweiterte Modifizierung), wobei das erweitert modifizierte Proteinfragment/Peptid im wesentlichen dieselbe Funktion bezüglich der Stimulation von T - Zellen aufweist, die das nicht modifizierte Proteinfragment/Peptid besitzt.

Abkürzungen: Die im Text verwendeten Abkürzungen sind durch die Regeln bestimmt, die von der IUPAC-IUB Kommission für biochemische Nomenklatur festgelegt worden sind (Biochemistry 11: 1726 (1972) und Biochem. J. 219: 345 (1984)). Folgende übliche Abkürzungen werden verwendet Ala = A = Alanin; Arg = R= Arginin; Asn =N = Asparagin; Cys = C = Cystein; Gln = Q = Glutamin; Glu = E = Glutaminsäure; Gly = G = Glycin; His = H = Histidin; Ile = I = Isoleucin; Leu = L = Leucin; Lys = K = Lysin; Met = M = Methionin; Phe = F = Phenylalanin; Pro = P = Prolin; Ser = S = Serin; Thr =T = Threonin; Trp = W = Tryptophan; Tyr = Y = Tyrosin und Val = V = Valin.

Vorteilhaft ist, wenn die Proteinfragmente, die an MHC-Klasse-II-Moleküle gebunden präsentiert werden, je nach Ende, Amino-Schutzgruppen oder CarboxylSchutzgruppen oder deren Varianten aufweisen.
Die Schutzgruppe oder deren Varianten für den N-Terminus kann bestehen aus: Alkyl-, Aryl-, Alkylaryl-, Aralkyl-, Alkylcarbonyl-oder Arylcarbonylgruppen mit 1 bis 10 Kohlenstoffatomen, bevorzugt sind Naphthoyl-, Naphthylacetyl-, Naphthylpropionyl-, Benzoylgruppe oder einer Acylgruppe mit 1 bis 7 Kohlenstoffatomen.
Die Schutzgruppe oder deren Varianten für den C-Terminus können bestehen aus: Einer Alkoxy-oder Aryloxygruppe mit 1 bis 10 Kohlenstoffatomen oder aus einer Aminogruppe.

### Verwendung von T-Zell-stimulierenden Proteinfragmenten als Medikament

Das erfindungsgemäße Verfahren umfasst weiterhin die Verwendung von einem Proteinfragment/Peptid, dessen Aminosäuresequenz bzw. ausgängliche Aminosäuresequenz nach dem erfindungsgemäßen Verfahren zur Identifikation T-Zellen-stimulierender Proteinfragmente gefunden wurde und welches nach dem erfindungsgemäßen Herstellungsverfahren produziert worden ist, zur Herstellung eines Medikaments zur Immunstimulation.

Am meisten bevorzugt ist die Verwendung eines Proteinfragmentes/Peptides, wobei die Immunstimulation eine Vakzinierung oder Desensibilisierung ist.
Die Vakzinierung besteht darin, daß als Antigen Proteine von Viren, Bakterien eukaryotischen Einzellem oder Vielzellem nach der Ermittlung ihrer Sequenz in Proteinfragmente aufgeteilt werden, die gemäß der Erfindung zu T-Zell-enthaltenden Suspensionen gegeben werden. Die positiven Ansätze, in denen sich ein T-Zellstimutierendes Proteinfragment befindet, werden als Ausgangspunkt für die Herstellung einer Vakzine verwendet

Die Desensibilisierung besteht darin, daß Proteinfragmente/Peptide ermittelt werden, die die unerwünschte, immunologische Reaktion auslösen. Anschließend werden die T-Zell-stimulierenden Proteinfragmente/Peptide bzw. die daraus entsprechend dem Herstellungsverfahren hergestellten Medikamente dem Patienten verabreicht. Der jeweils gewünschte Effekt (Stimulation oder Desensibilisierung) wird über die Art und den Ort der Anwendung sowie die Dosis (z.B. Hochdosis-oder Niedrigdosistoleranzinduktion) und die begleitende Verabreichung beispielweise stimulierender oder tolerisierender Zytokine oder ähnlicher immunmodulatorisch aktiver Medikamente erreicht bzw. verstärkt. Proteinfragmente, die nicht nach diesem erfindungsgemäßen Verfahren aufgefunden worden sind, wurden bereits erfolgreich als Medikamente eingesetzt, so z.B. bei der Vakzinierung von Rindern gegen Maul- und Klauenseuche (Collen et al.; J Immunol 1991; 146:749-755). Das in unserem Beispiel identifizierte Peptid wurde parallel durch konventionelle Technik von einer anderen Gruppe gefunden und befindet sich als Vakzine in Erprobung (Diamond et al. Blood 1997; 5:1751-1767).

### Beispiele

### Beispiel 1

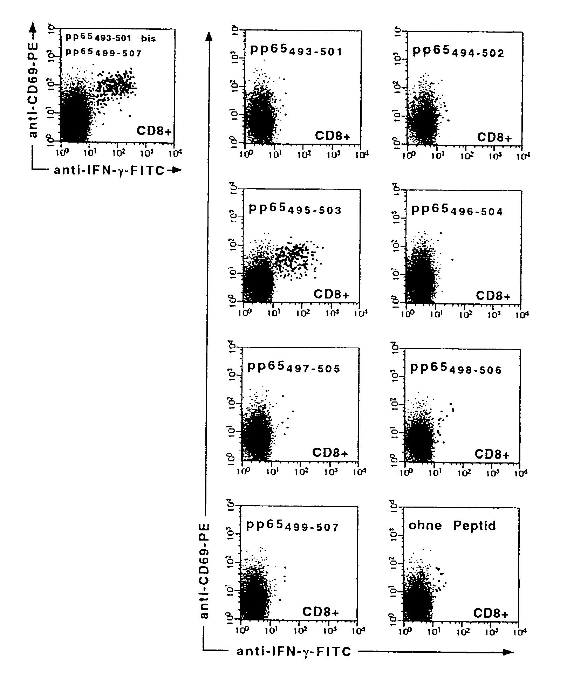

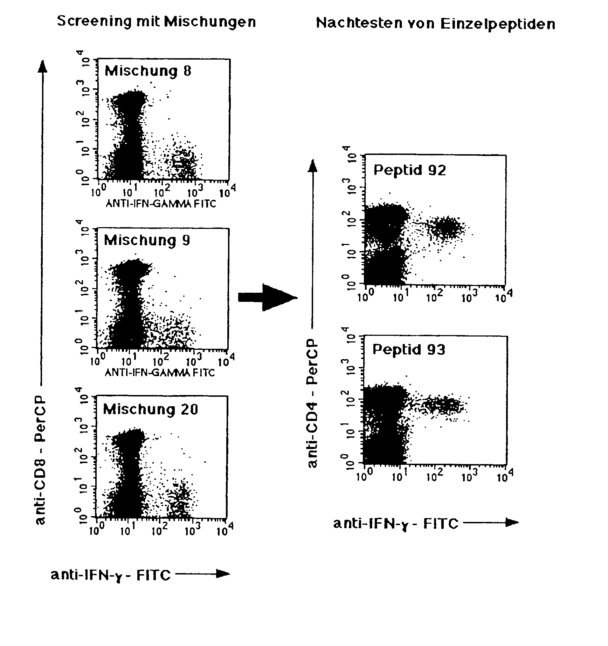

### (Siehe Abbildung 1/2).

Mononukleäre Zellen wurden aus dem durch venöse Punktion gewonnenen peripheren Blut einer HLA-typisierten Patientin präpariert, welche das MHC-Klasse-I Allel HLA-A*0201 besaß. Die Patientin besaß außerdem Antikörper gegen das humane Cytomegalie-Virus. Die nach Standardmethode präparierten Zellen wurden für sechs Stunden unter optimierten Bedingungen mit den unten angegebenen Peptiden inkubiert. Diese stellen Bruchstücke eines aus der Literatur bekannten Proteinfragmentes des pp 65-Proteins des humanen Cytomegalie-Virus (Swiss-Prot PO6725) von 15 Aminosäuren Länge dar (Ala Arg Asn Leu Val Pro Met Val Ala Thr Val Gln Gly Gln Asn, pp65₄₉₃₋₅₀₇). Dieses Proteinfragment ist bekannt dafür, daß es in der Bulk-Kultur HLA-A2 restringierte, zytotoxische T-Zellen induzieren kann, also ein mit HLA-A2 präsentiertes T-Zell-Epitop enthält (M. R. WILLS et al. (1996) J. Virol. Vol. 70, pp 7569-5779). Die Länge von 9 Aminosäuren für die zu testende Bruchstücke wurde gewählt, da dieses die typische Länge von Epitopen ist, welche mit MHC-Klasse-I-Molekülen präsentiert werden (H. G. RAMMENSEE et al. (1995) Immunogenetics, Vol 41, pp 178-228). Die verwendeten Peptide überlappen sich um jeweils 8 Aminosäuren und stellen somit alle möglichen Bruchstücke dieser Länge dar. Die Peptide wurden als Mischung aus allen Peptiden oder einzeln eingesetzt. Die Peptidkonzentration im gezeigten Beispiel betrug 1 µg/ml je Peptid.
Folgenden Peptide wurden eingesetzt:
1) Ala Arg Asn Leu Val Pro Met Val Ala
2) Arg Asn Leu Val Pro Met Val Ala Thr
3) Asn Leu Val Pro Met Val Ala Thr Val
4) Leu Val pro Met Val Ala Thr Val Gln
5) Val pro Met Val Ala Thr Val Gln Gly
6) pro Met Val Ala Thr Val Gln Gly Gln
7) Met Val Ala Thr Val Gln Gly Gln Asn

Die Inkubation mit der Mischung aus allen Peptiden (Abbildung: Diagramm oben links) sowie Peptid 3 allein (Abbildung: Diagramm in der Mitte, zweites von oben) führten zur Produktion von IFN-γ in T-Zellen, welches durch Messung am Durchflußzytometer auf Einzel-Zellebene (J. L. PICKER et al., (1995) Blood, Vol 86, pp 1408-1419) nachgewiesen wurde, Keines der anderen einzeln getesteten Peptide hatte diesen Effekt. Eine in der Literatur veröffentlichte Untersuchung identifizierte exakt das gleiche Epitop innerhalb des gleichen Proteinsegments durch konventionelle Methoden und bestätigt unser Ergebnis eindeutig (D. J. DIAMOND et al. (1997) Blood, Vol 90, pp 1751-1767).

### Legende zur Abbildung 1/2:

Detektion von intrazellulär vorliegendem Interferon-γ in CD8⁺ T-Lymphozyten nach Stimulation mit der Mischung aus den 7 angegebenen Peptiden (Oben, ganz links) beziehungsweise den einzelnen Peptiden, pp65₄₉₃₋₅₀₁ bis pp65₄₉₉₋₅₀₇. Der Marker CD69 wurde als Aktivierungsmarker verwendet. Die Darstellung ist auf CD3⁺/CD8⁺ Ereignisse eingegrenzt, angegeben ist die mittlere Fluoreszenzintensität.

### Beispiel 2

### (Siehe Abbildung 2/2)

Mononukleäre Zellen wurden aus dem durch venöse Punktion gewonnenen peripheren Blut einer HLA-typisierten Patientin präpariert, welche das MHC-Klasse-II Allel HLA-DR11 besaß. Die Patientin besaß außerdem Antikörper gegen das humane Cytomegalie-Virus. Die nach Standardmethode präparierten Zellen wurden für sechs Stunden unter optimierten Bedingungen mit Mischungen aus 11 oder 12 jeweils 15-Aminosäuren-langen Peptiden mit jeweils 11 Überlappungen entsprechend der Sequenz des pp65 Matrix Phosphoproteins (Swiss-Prot PO6725) inkubiert (insgesamt 138 Peptide). Die Peptidkonzentration betrug 1 µg/ml je Peptid. Drei der insgesamt 24 Mischungen stimulierten eindeutig CD4⁺ T-Zellen. Aufgrund der Versuchsanordnung (Vorkommen bestimmter Peptide in bestimmten Mischungen) ließen sich damit eindeutig 2 Peptide identifizieren, welche für diese Stimulation verantwortlich waren. Dieses Ergebnis wurde durch die Stimulation mit den jeweils einzelnen Peptiden unter ansonsten gleichen Bedingungen bestätigt. Die identifizierten Peptide waren die benachbarten Peptide pp65₃₆₅₋₃₇₉ und pp65₃₆₉₋₃₈₃. Diese Sequenzen decken sich weitgehend mit folgenden in der Literatur beschriebenen HLA-DR11 präsentierten Peptidesequenzen, welche auf konventionelle Weise als T-Zellen-stimulierende Sequenzen identifiziert wurden: pp65₃₆₁₋₃₇₆ und pp65₃₆₉₋₃₈₄ (Khattab et al. (1998) Joumal of Medical Virology, Vol. 52, pp68-76), d.h, die stimulierenden Peptide finden sich innerhalb des Abschnitts definiert durch die Aminosäuren 361 und 384. Eine weitere Einengung der Epitopsequenz auf die zu postulierende Länge von 11 Aminosäuren ist noch nicht erfolgt.

### Legende zur Abbildung 2/2

Detektion von intrazellulär vorliegendem Interferon-γ in CD3⁺/CD8⁻ (links) nach Stimulation mit den Peptidmischungen 8, 9, und 20, bzw. CD3⁺/CD4⁺ T-Lymphozyten (rechts) nach Stimulation mit den einzelnen Peptiden pp65₃₆₅₋₃₇₉ und pp65₃₆₉₋₃₈₃. Beim Screening (rechts) wurden Peptidmischungen verwendet und CD3 und CD8 als T-Zellmarker. Da die INF-γ⁺ Populationen links CD3⁺/CD8⁻ sind wurde beim Nachtesten der Marker CD4 verwendet. Die Stimulierten T-Zellen sind eindeutig CD4⁺. Dargestellt sind ausschließlich CD3⁺ Zellen, angegeben ist die mittlere Fluoreszenzintensität.

## Patentansprüche

1. Verfahren zum Identifizieren von T-Zell-stimulierenden Proteinfragmenten, die folgenden Schritte umfassend:
a) Ermitteln der Aminosäuresequenz eines Antigens, welches ein Protein oder Peptid ist,
b) Unterteilen der gefundenen Aminosäuresequenz des Antigens in Proteinfragmente,
c) Synthetisieren von mindestens einem Proteinfragment mit einer Länge von B bis 30 Aminosäuren oder Spalten der Aminosäuresequenz des Antigens zu mindestens einem Proteinfragment mit einer Länge von 8 bis 30 Aminosäuren,
dabei ist das Proteinfragment eine Teilsequenz der ermittelten Aminosäuresequenz des Antigens.
d) Inkubieren einer T-Zellen enthaltenden Suspension mit dem oder den Proteinfragmenten in Versuchsansätzen,
e) Identifizieren
(i) von mindestens einem T-Zell-Zytokin,
das durch das oder die Proteinfragmente induziert und in den T-Zellen synthetisiert wurde,
dabei liegen das oder die T-Zell-Zytokine intrazellulär oder an die Zellmembran gebunden vor,
und / oder
(ii) von mindestens einem aufgrund der T-Zellstimulation durch das oder die Proteinfragmente exprimierten oder in seiner Expression gesteigerten Aktivierungsmarker, der durch das oder die Proteinfragmente induziert oder in seiner Expression gesteigert wurde und in den T-Zellen exprimiert wird,
dabei kann der Aktivierungsmarker intrazellulär vorliegen oder auf der Zelloberfläche exprimiert sein
dabei werden das oder die T-Zell-Zytokine oder Aktivierungsmarker durchflußzytometrisch identifiziert, und
f) Zuordnen der Versuchsansätze, bei denen T-Zellen stimuliert wurden und diese T-Zell-Stimulation durch das Identifizieren von einem T-Zell-Zytokin oder mehreren T-Zell-Zytokinen und / oder einem oder mehreren Aktivierungsmarkern erkannt wurde, zu der oder den Aminosäuresequenzen der Proteinfragmente, welche mit den T-Zellen inkubiert wurden,
**dadurch gekennzeichnet,**
**daß** die Inkubationszeit so lang ist,
**daß** das oder die Proteinfragmente von den MHC Molekülen,
die sich auf der Zelloberfläche befinden,
ausreichend aufgenommen werden,
wobei die Aufnahme dann ausreichend ist,
wenn eine eindeutige Identifizierung stimulierter T-Zellen möglich ist,
und,
**daß** die Inkubationszeit der T-Zellen enthaltenden Suspension mit dem oder den Proteinfragmenten so kurz ist,
**daß** eine Selektion und Proliferation, die mit einer gezielten Eliminierung bestimmter T-Zellen einhergeht, nicht erfolgt.

2. Verfahren zum Identifizieren von T-Zell-stimulierenden Proteinfragmenten nach Anspruch 1, wobei das Identifizieren von mindestens einem T-Zell-Zytokin oder Aktivierungsmarker auf der Einzelzell-Ebene erfolgt.

3. Verfahren zum Identifizieren von T-Zell-stimulierenden Proteinfragmenten nach einem der vorherigen Ansprüche, wobei die T-Zellen enthaltende Suspensionen Zellen enthalten,
die das Proteinfragment im wesentlichen an MHC-Klasse-I oder Klasse-II-Moleküle gebunden präsentieren.

4. Verfahren zum Identifizieren von T-Zell-stimulierenden Proteinfragmenten nach einem der vorherigen Ansprüche, wobei das Proteinfragment bei der Klasse I restringierten Präsentation 9 bis 11 Aminosäuren umfaßt und das Proteinfragment bei der Klasse II restringierten Präsentation mindestens 11 Aminosäuren umfaßt.

5. Verfahren zum Identifizieren von T-Zell-stimulierenden Proteinfragmenten nach einem der vorherigen Ansprüche, wobei die T-Zellen enthaltende Suspension eine Suspension ist aus Vollblut, peripheren weißen Blutzellen (PWBC), Milzzellen, Thymuszellen, Knochenmark, Liquor und / oder aus Lymphknotenzellen.

6. Verfahren zum Identifizieren von T-Zell-stimulierenden Proteinfragmenten nach einem der vorherigen Ansprüche, wobei die T-Zellen enthaltende Suspension aus den Patienten, die therapiert werden sollen, aus Spendern oder aus Tieren stammen.

7. Verfahren zum Identifizieren von T-Zell-stimulierenden Proteinfragmenten nach einem der vorherigen Ansprüche, wobei die Antigene, welche Proteine oder Peptide sind aus mehrzelligen Eukaryoten, aus Zellen, Zellkulturen und / oder Geweben von Spendern oder Patienten stammen.

8. Verfahren zum Identifizieren von T-Zell-stimulierenden Proteinfragmenten nach einem der vorherigen Ansprüche, wobei die T-Zell-Zytokine vom Typ Interteron-γ, TNF-α oder Interleukin 2 sind.

9. Verfahren zum Identifizieren von T-Zell-stimulierenden Proteinfragmenten nach einem der vorherigen Ansprüche weiterhin umfassend das Herstellen von identifizierten Proteinfragment/Peptid mit der Festphasenmethode, der Flüssigphasenmethode oder mittels der Proteinbiosynthese in einem Wirt.

10. Verfahren zum Identifizieren von T-Zell-stimulierenden Proteinfragmenten nach Anspruch 9, wobei das Proteinfragment/Peptid Insertionen, Deletionen oder Substituierungen (Modifikationen) aufweist, wobei eine, zwei, drei oder mehrere Aminosäuren ausgetauscht, deletiert oder inserieret sind, und wobei das modifizierte Proteinfragment/Peptid im wesentlichen dieselbe Funktion bezüglich der Stimulation von T - Zellen aufweist, die das nicht modifizierte Proteinfragment/Peptid besitzt.

11. Verfahren zum Identifizieren von T-Zell-stimulierenden Proteinfragmenten nach Anspruch 9 oder 10, wobei das Proteinfragment / Peptid am N-terminalen und / oder C-terminalen Ende mindestens eine weitere natürliche oder nichtnatürliche Aminosäure und / oder eine Schutzgruppe besitzt (erweiterte Modifizierung), und
wobei das erweitert modifizierte Proteinfragment/Peptid im wesentlichen dieselbe Funktion bezüglich der Stimulation von T - Zellen aufweist, die das nicht modifizierte Proteinfragment/Peptid besitzt.

12. Verfahren zur Identifizierung von T-Zell-stimulierenden Proteinfragmenten nach einem oder mehreren der Ansprüche 9-11, wobei die Proteinfragmente/Peptide zur Herstellung eines Medikaments zur Immunstimulation geeignet sind.

13. Verfahren zur Identifizierung von T-Zell-stimulierenden Proteinfragmenten nach Anspruch 12, wobei die Immunstimulation eine Vakzinierung oder Desensibilisierung ist.

## Claims

1. A method for the identification of T-cell stimulating protein fragments comprising the following steps:
a) establishing the amino acid sequence of an antigen which is a protein or a peptide;
b) subdividing the detected amino acid sequence of said antigen into protein fragments;
c) synthesizing at least one protein fragment having a length of from 8 to 30 amino acids, or cleaving the amino acid sequence of said antigen into at least one protein fragment having a length of from 8 to 30 amino acids, wherein said protein fragment is a subsequence of the established amino acid sequence of said antigen;
d) incubating a suspension containing T cells with the protein fragment or fragments in different experimental runs;
e) identifying of
(i) at least one T cell cytokine which has been induced by the protein fragment or fragments and synthesized in the T cells, wherein the T cell cytokine or cytokines remain within the cell or are bound to the cell membrane; and/or
(ii) at least one activation marker expressed or expression-enhanced due to the T cell stimulation by the protein fragment or fragments which has been induced or expression-enhanced by the protein fragment or fragments and which is expressed in the T cells, wherein said activation marker can be present within the cell or expressed on the cellular surface;
wherein said T cell cytokine or cytokines or activation markers are identified by flow cytometry; and
f) assigning the experimental runs in which T cells have been stimulated and such stimulation has been recognized by the identification of one or more T cell cytokines and/or one or more activation markers, to the amino acid sequence or sequences of said protein fragments which had been incubated with the T cells;
**characterized in that** the incubation time is sufficiently long so that the protein fragment or fragments are sufficiently taken up by the MHC molecules present on the cellular surface, said taking up being sufficient when an unambiguous identification of stimulated T cells is possible; and
that the incubation time of the suspension containing T cells with the protein fragment or fragments is sufficiently short so that selection and proliferation accompanied by the specific elimination of particular T cells do not occur.

2. The method for the identification of T-cell stimulating protein fragments according to claim 1, wherein said identification of at least one T cell cytokine or activation marker is made on the individual cell level.

3. The method for the identification of T-cell stimulating protein fragments according to any of the preceding claims, wherein said suspensions containing T cells contain cells which present the protein fragment essentially in a state bound to MHC class I or class II molecules.

4. The method for the identification of T-cell stimulating protein fragments according to any of the preceding claims, wherein the protein fragment in the class I restricted presentation comprises from 9 to 11 amino acids, and the protein fragment in the class II restricted presentation comprises at least 11 amino acids.

5. The method for the identification of T-cell stimulating protein fragments according to any of the preceding claims, wherein said suspension containing T cells is a suspension of whole blood, peripheral white blood cells (PWBC), splenocytes, thymocytes, bone marrow, cerebrospinal fluid and/or lymph node cells.

6. The method for the identification of T-cell stimulating protein fragments according to any of the preceding claims, wherein said suspension containing T cells is derived from the patients to be subjected to therapy, from donors or from animals.

7. The method for the identification of T-cell stimulating protein fragments according to any of the preceding claims, wherein the antigens, i.e., proteins or peptides, are derived from polycellular eukaryotes, cells, cell cultures and/or tissues of donors or patients.

8. The method for the identification of T-cell stimulating protein fragments according to any of the preceding claims, wherein the T cell cytokines are of the types interferon-γ, TNF-α or interleukin 2.

9. A process for the identification of T-cell stimulating protein fragments according to any of the preceding claims, further comprising the preparation of identified protein fragments/peptides by the solid phase method, liquid phase method or by protein biosynthesis in a host.

10. The process for the identification of T-cell stimulating protein fragments according to claim 9, wherein said protein fragment/peptide contains insertions, deletions or substitutions (modifications) wherein one, two, three or more amino acids have been exchanged, deleted or inserted, wherein said modified protein fragment/peptide has essentially the same function with respect to the stimulation of T cells as the non-modified protein fragment/peptide.

11. The process for the identification of T-cell stimulating protein fragments according to claim 9 or 10, wherein said protein fragment/peptide contains at least one additional naturally occurring or not naturally occurring amino acid and/or a protecting group at the N-terminal and/or C-terminal end (extended modification), wherein the extendedly modified protein fragment/peptide has essentially the same function with respect to the stimulation of T cells as the non-modified protein fragment/ peptide.

12. The process for the identification of T-cell stimulating protein fragments according to one or more of claims 9 to 11, wherein said protein fragments/peptides are suitable for the preparation of a medicament for immune stimulation.

13. The process for the identification of T-cell stimulating protein fragments according to claim 12, wherein said immune stimulation is a vaccination or desensitization.

## Revendications

1. Procédé pour identifier des fragments de protéine stimulant les lymphocytes T, comprenant les étapes suivantes :
a) détermination de la séquence d'acides aminés d'un antigène, qui est une protéine ou un peptide,
b) subdivision de la séquence d'acides aminés trouvée de l'antigène en fragments de protéine,
c) synthèse d'au moins un fragment de protéine ayant une longueur de 8 à 30 acides aminés ou cassure de la séquence d'acides aminés de l'antigène en au moins un fragment de protéine ayant une longueur de 8 à 30 acides aminés, le fragment de protéine étant alors une séquence partielle de la séquence d'acides aminés, déterminée, de l'antigène,
d) incubation d'une suspension contenant des lymphocytes T avec le ou les fragments de protéine ou des préparations d'essai,
e) identification
(i) d'au moins une cytokine de lymphocyte T,
qui a été induite par le ou les fragments de protéine et synthétisée dans les lymphocytes T,
la ou les cytokines de lymphocyte T se présentant alors liées intracellulairement ou à la membrane des cellules, et/ou
(ii) d'au moins un marqueur d'activation exprimé ou amplifié dans son expression par le ou les fragments de protéine, en raison de la stimulation de lymphocytes T, marqueur d'activation qui a été induit ou amplifié dans son expression par le ou les fragments de protéine et est exprimé dans les lymphocytes T,
le marqueur d'activation pouvant alors être présent intracellulairement ou être exprimé sur la surface des lymphocytes,
la ou les cytokines de lymphocyte T ou le ou les marqueurs d'activation étant alors identifiés par cytométrie de flux, et
f) association des préparations d'essai, pour lesquelles des lymphocytes T ont été stimulés et cette stimulation de lymphocytes T a été décelée par l'identification d'une cytokine de lymphocyte T ou de plusieurs cytokines de lymphocyte T et/ou d'un ou de plusieurs marqueurs d'activation, à la ou aux séquences d'acides aminés des fragments de protéine, qui ont été incubés avec les lymphocytes T,
**caractérisé**
**en ce que** la durée d'incubation est suffisamment longue
pour que le ou les fragments de protéine soient absorbés suffisamment par les molécules de MHC,
qui se trouvent sur la surface des cellules,
l'absorption étant suffisante
lorsqu'une identification non équivoque de lymphocytes T stimulés est possible,
et
**en ce que** la durée d'incubation de la suspension contenant des lymphocytes T, avec le ou les fragments de protéine, est suffisamment brève
pour qu'une sélection et une prolifération, qui interviennent avec une élimination ciblée de lymphocytes T déterminés, n'aient pas lieu.

2. Procédé pour identifier des fragments de protéine stimulant les lymphocytes T selon la revendication 1, dans lequel l'identification d'au moins une cytokine de lymphocyte T ou d'au moins un marqueur d'activation s'effectue dans le plan d'une cellule individuelle.

3. Procédé pour identifier des fragments de protéine stimulant les lymphocytes T selon l'une des revendications précédentes, dans lequel les suspensions contenant des lymphocytes T contiennent des cellules qui présentent le fragment de protéine lié essentiellement à des molécules de MHC de classe I ou de classe II.

4. Procédé pour identifier des fragments de protéine stimulant les lymphocytes T selon l'une des revendications précédentes, dans lequel le fragment de protéine, dans le cas de la présentation restreinte par la classe I, comprend 8 à 11 acides aminés et le fragment de protéine, dans le cas de la présentation restreinte par la classe II, comprend au moins 11 acides aminés.

5. Procédé pour identifier des fragments de protéine stimulant les lymphocytes T selon l'une des revendications précédentes, dans lequel la suspension contenant des lymphocytes T est une suspension de sang total, de globules blancs du sang périphérique (PWBC), de cellules de la rate, de cellules du thymus, de moelle osseuse, de la fraction sérum-fibrines du sang et/ou de cellules de ganglions lymphatiques.

6. Procédé pour identifier des fragments de protéine stimulant les lymphocytes T selon l'une des revendications précédentes, dans lequel la suspension contenant des lymphocytes T provient des patients, qui doivent être traités, de donneurs ou d'animaux.

7. Procédé pour identifier des fragments de protéine stimulant les lymphocytes T selon l'une des revendications précédentes, dans lequel les antigènes, qui sont des protéines ou des peptides, proviennent d'eucaryotes multicellulaires, de cellules, de cultures cellulaires et/ou de tissus de donneurs ou de patients.

8. Procédé pour identifier des fragments de protéine stimulant les lymphocytes T selon l'une des revendications précédentes, dans lequel les cytokines de lymphocyte T sont du type interféron-y, TNF-α ou interleukine-2.

9. Procédé pour identifier des fragments de protéine stimulant les lymphocytes T selon l'une des revendications précédentes, comprenant, en outre, la production de fragment de protéine/peptide identifié avec la méthode en phase solide, la méthode en phase liquide ou au moyen de la biosynthèse de protéines dans un hôte.

10. Procédé pour identifier des fragments de protéine stimulant les lymphocytes T selon la revendication 9, dans lequel le fragment de protéine/le peptide présente des insertions, des délétions ou des substitutions (modifications), un, deux, trois acides aminés, ou plus, étant changés, supprimés ou insérés, et le fragment de protéine/le peptide, modifié, présentant sensiblement la même fonction, en ce qui concerne la stimulation de lymphocytes T, que celle que possède le fragment de protéine/le peptide non modifié.

11. Procédé pour identifier des fragments de protéine stimulant les lymphocytes T selon la revendication 9 ou 10,
dans lequel le fragment de protéine/le peptide possède, à l'extrémité N-terminale et/ou C-terminale, au moins un acide aminé supplémentaire, naturel ou non naturel, et/ou un groupe protecteur (modification élargie), et
dans lequel le fragment de protéine/le peptide, ayant fait l'objet d'une modification élargie, présente sensiblement la même fonction, en ce qui concerne la stimulation de lymphocytes T, que celle que possède le fragment de protéine/le peptide non modifié.

12. Procédé pour identifier des fragments de protéine stimulant les lymphocytes T selon une ou plusieurs des revendications 9 à 11, dans lequel les fragments de protéine/les peptides conviennent pour la production d'un médicament d'immunostimulation.

13. Procédé pour identifier des fragments de protéine stimulant les lymphocytes T selon la revendication 12, dans lequel l'immunostimulation est une vaccination ou une désensibilisation.
